# EUROPEAN PATENT SPECIFICATION

(11) **EP 3 462 932 B1**
(45) Date of publication and mention of the grant of the patent: **15.04.2020**
(21) Application number: 17724839.0
(22) Date of filing: 25.05.2017
(51) Int. Cl.: A24F 47/00

(54) **HEATER AND WICK ASSEMBLY FOR AN AEROSOL GENERATING SYSTEM**
HEIZELEMENT UND DOCHTANORDNUNG FÜR EIN AEROSOLERZEUGUNGSSYSTEM
ÉLÉMENT CHAUFFANT ET ENSEMBLE MÈCHE POUR UN SYSTÈME DE GÉNÉRATION D'AÉROSOL

(30) Priority: 31.05.2016 EP 16172208
(43) Date of publication of application: 10.04.2019
(73) Proprietor: Philip Morris Products S.A., 2000 Neuchâtel (CH)
(72) Inventor: BATISTA, Rui Nuno, 1110 Morges (CH)
(74) Representative: Lupini, Stephen Antony
(86) International application number: PCT/EP2017/062719
(87) International publication number: WO 2017/207415

(56) References cited:
- EP-A1- 2 340 729
- WO-A1-2016/033891
- US-A1- 2013 192 619
- US-A1- 2014 270 730
- US-A1- 2015 083 147

## Description

The present invention relates to heater and wick assemblies for aerosol generating systems that incorporate a heating element and capillary body. The disclosure also relates to methods of producing such heater and wick assemblies.

Electrically heated smoking systems that are handheld and operate by heating a liquid aerosol-forming substrate in a capillary wick are known in the art. For example, WO2009/132793 describes an electrically heated smoking system comprising a shell and a replaceable mouthpiece. The shell comprises an electric power supply and electric circuitry. The mouthpiece comprises a liquid storage portion and a capillary wick having a first end and a second end. The first end of the wick extends into the liquid storage portion for contact with liquid therein. The mouthpiece also comprises a heating element for heating the second end of the capillary wick, an air outlet, and an aerosol-forming chamber between the second end of the capillary wick and the air outlet. The heating element is typically a coil of wire that is wound around the wick. When the shell and mouthpiece are engaged, the heating element is in electrical connection with the power supply via the circuitry, and a flow route for air is defined from at least one air inlet to the air outlet via the aerosol-forming chamber. In use, liquid is transferred from the liquid storage portion towards the heating element by capillary action in the wick. Liquid at the second end of the capillary wick is vaporised by the heating element. The supersaturated vapour created, is mixed and carried in the air-flow from the at least one air inlet to the aerosol-forming chamber. In the aerosol-forming chamber, the vapour condenses to form an aerosol, which is carried towards the air outlet into the mouth of a user.

WO 2016/033891 A1 discloses a heating module of an electronic cigarette atomizer comprising; a ceramic rod having a hollow microporous structure, a heating wire wound around the ceramic rod and a pair of conducting elements connected to the two ends of the heating wire respectively.

The specific characteristics of the wick and heater assembly are important for achieving the required functional performance. Therefore, the ability to accurately and consistently produce wick and heater assemblies is important in maintaining consistent performance between different aerosol generating systems of the same type. For example, in heater assemblies having a heater coil, the heater coils should be produced with the same dimensions and have the same number and pitch of coil turns to reduce product-to-product variability. In known systems, the manufacture of the heater coil and assembly of the heater may require a high number of manufacturing steps some of which may need to be carried out manually by a skilled operator, for example where the heater coil requires connection to electrical contacts by welding. Existing heater assemblies may also be fragile and thus require delicate handling during manufacture, transport and storage to avoid damage or deformation which may otherwise cause changes to its physical or electrical characteristics.

It would be desirable to provide a heater and wick assembly for an aerosol generating system which is robust and which can be manufactured consistently and more simply.

According to a first aspect of the present invention, there is provided a heater and wick assembly for an aerosol generating system, the assembly comprising: a capillary body; a heating element arranged on an outer surface of the capillary body; a pair of spaced apart electrical contacts fixed around the capillary body and coupled with the heating element, and a support member extending along at least part of the length of the capillary body.

The electrical contacts are preferably positioned over the heating element. By fixing the electrical contacts around the capillary body and over the heating element, the electrical contacts may secure the heating element to the outer surface of the capillary body. That is, the electrical contacts may hold at least part of the heating element in place on the outer surface of the capillary body. With this arrangement, the electrical contacts may secure the heating element to the capillary body as well as providing an electrical connection by which the heating element may be connected to a source of electrical energy. Advantageously, this arrangement requires fewer manufacturing steps than existing systems in which the ends of the heater element are manually connected to the electrical contacts, for example by welding. It may also allow the heater and wick assembly to be manufactured on an automated assembly line, so such devices can be manufactured more quickly with high repeatability.

Preferably, at least one of the electrical contacts is dimensioned such that there is a frictional fit between an inner surface of that electrical contact and the outer surface of the capillary body. Providing such a frictional fit may allow the electrical contact to be secured on the capillary body without the need for additional fastening means or fastening steps. Preferably, each electrical contact is dimensioned such that there is a frictional fit between the inner surface of the electrical contact and the outer surface of the capillary body.

The electrical contacts may be fixed to the outer surface of the capillary body using an adhesive or similar fastening means.

The electrical contacts may extend around at least part of the circumference of the capillary body. The capillary body may be compressible. At least one of the electrical contacts may be dimensioned such that there is an interference fit between the electrical contact and the capillary body. That is, the electrical contact may be dimensioned such that its inner surface defines an internal space having a diameter which is less than the outer diameter of the capillary body so the capillary body is compressed by the electrical contact to secure the electrical contact to the capillary body. The capillary body may be compressible and at least one of the electrical contacts may extend around at least part of the circumference of the capillary body and be dimensioned such that there is an interference fit between the electrical contact and the capillary body. This may help to ensure that the heating element is securely fixed to the capillary body by the electrical contact without the need for adhesive or additional fixation steps, such as soldering or welding. It may also help to ensure a reliable electrical connection between the electrical contact and the heating element. In such embodiments, both of the electrical contacts may extend around at least part of the circumference of the capillary body and each may be dimensioned such that there is an interference fit between the electrical contact and the capillary body.

The electrical contacts may extend around only part of the circumference of the capillary body. The electrical contacts preferably extend around more than 50 percent of the circumference of the capillary body. This may result in a more secure fixation of the electrical contacts to the capillary body relative to examples in which the electrical contacts extend around less than 50 percent of the circumference of the capillary body. It may also help to ensure a reliable electrical connection between the electrical contact and the heating element.

One or both of the electrical contacts may extend around substantially the entire circumference of the capillary body. At least one of the electrical contacts may circumscribe the capillary body. In such embodiments, the electrical contact may be ring shaped. Preferably, both electrical contacts circumscribe the capillary body. This may result in a more secure fixation of the electrical contacts to the capillary body relative to examples in which the electrical contacts extend around less than the entire circumference of the capillary body. It may also help to ensure a reliable electrical connection between the electrical contact and the heating element irrespective of the specific arrangement of the heating element on the outer surface of the capillary body and without restricting the arrangement of the heating element to ensure contact between the electrical contacts and the heating element.

Both electrical contacts may circumscribe the capillary body and be dimensioned such that there is an interference fit between the electrical contacts and the capillary body.

The electrical contacts may be rigid. This may result in a more robust assembly than one in which the electrical contacts are flexible. The electrical contacts may be rigid, extend around more than 50 percent of the circumference of the capillary body and be dimensioned such that there is a frictional fit between the capillary body and the electrical contacts. This may allow the electrical contacts simply to be clipped around the capillary body during assembly.

The electrical contacts may each comprise a ring of rigid material, such as a metallic ring. This may provide an electrical contact with high mechanical resistance and reliable electrical connection to the heating element. It may also enable the heater and wick assembly to be connected to a supply of electrical energy in an aerosol generating device by snap fitting the electrical contacts into a retaining clip in the device.

Where the electrical contacts extend around the circumference of the capillary body, each electrical contact may be formed from a bent sheet of material, the opposed ends of which are connected together at a joint. For example, the opposed ends may be connected together at the joint by snap fitting or clamping. This may provide a robust assembly which does not require welding.

Where the electrical contacts extend around the circumference of the capillary body, the opposed ends of each electrical contact may be co-operatively shaped such that the joint is non-linear or extends along an oblique line. In this context, the term "oblique line" means that the joint extends along a line which is nonparallel to the longitudinal axis of the capillary body. By having a joint which is non-linear or extending along an oblique line, relative movement between the opposed ends of each electrical contact in the longitudinal direction of the capillary body may be prevented or minimised.

The electrical contacts may be flexible. For example, the electrical contacts may be formed from a flexible sheet of electrically conductive material, such as a metal foil. In such embodiments, the electrical contacts may be secured to the outer surface of the capillary body using an adhesive or similar, or extend around the entire circumference of the capillary body such that there is a frictional fit between the capillary body and the electrical contacts.

In any of the above embodiments, the heating element may comprise a coil of electrically resistive wire wound around the capillary body. In such embodiments, the coil of electrically resistive wire may be wound around the capillary body along the entire length of the capillary body.

The capillary body may be any suitable shape. The capillary body may be elongate. The pair of electrical contacts may be spaced apart in a length direction of the capillary body. In such embodiments, the pair of electrical contacts may be positioned at any location along the length of the capillary body. For example, the pair of electrical contacts may comprise a first electrical contact at or adjacent to a first end of the capillary body and a second electrical contact at any other location, such as at a midpoint along the length of the capillary body. The pair of electrical contacts may comprise a first electrical contact at or adjacent to a first end of the capillary body and a second electrical contact at or adjacent to the second end of the capillary body.

The heater and wick assembly comprises a support member extending along at least part of the length of the capillary body. The support member is stronger and stiffer than the capillary body. With this arrangement, the support member may increase the strength and rigidity of the heater and wick assembly to improve robustness and ease of handling. In manufacturing operations in which individual heater and wick assemblies are cut from a multi-length heater and wick assembly, the support member may result in improved accuracy of the cutting operation. This may lead to greater repeatability and consistency between different heater and wick assemblies.

The support member may be formed from a single, unitary component or from a plurality of components connected together.

Preferably, the support member is located within the capillary body. The support member may extend through the core of the capillary body. The support member may be surrounded by the capillary body. The support member may be circumscribed by the capillary body. The presence of the rigid support member may reduce the overall radial compressibility of the capillary body, thus helping to ensure a tight fit between the electrical contacts and the heating element. The support member may be arranged on an outer surface of the capillary body.

The support member is preferably a rigid support member.

The support member may extend along only part of the length of the capillary body. In some examples, the support member extends along substantially the entire length of the capillary body.

The support member may have any suitable cross-sectional area. In some examples, the support member has a cross-sectional area which is less than about 3 to 21 percent of the total cross sectional area of the capillary body, more preferably between about 4 to 16 percent of the cross-sectional area of the capillary body.

The support member may have any suitable cross-sectional shape. For example, the support member may have a planar, circular, oval, square, rectangular, triangular, or similar cross-sectional shape. The support member may have a solid cross-sectional area. The support member may have a hollow cross-sectional area.

In some examples, the support member may comprise a central portion and a plurality of transverse ribs. This cross-sectional shape may result in a support member having a suitable rigidity without occupying a large amount of space within the capillary body and thus significantly reducing the wicking ability of the capillary body. The plurality of transverse ribs may comprise a plurality of radially extending ribs. For example, the support member may comprise a central portion and three or more radially extending ribs. This may provide support member which is resistant to bending in all transverse directions.

In some examples, the support member is formed from an electrical insulative material. This may prevent the core component from impacting on the electrical performance of the heating element if it comes into contact with the heating element or the electrical contacts. The support member may be formed from an electrically conductive material.

The electrical contacts are fixed around the capillary body and are coupled with the heating element. Thus, the electric contacts may allow the heating element to be electrically connected to a supply of electrical energy. Preferably, the electrical contacts have a lower electrical resistance than the electrical resistance of the heating element, to prevent damage to the electrical contacts when the heating element is energized. In such examples, the electrical contacts may each have a larger cross-sectional area than the cross-sectional area of the heating element to which it is electrically connected. The electrical contacts may be formed from a material having a lower resistivity than a material from which the heating element is formed. Suitable materials for forming the electrical contacts include aluminium, copper, zinc, silver, stainless steel, such as austenitic 316 stainless steel and martensitic 440 and 420 stainless steel, and alloys thereof.

The heating element may be a coil of electrically resistive wire. The heating element may be formed by stamping or etching a sheet blank that can be subsequently wrapped around a wick. Preferably, the heating element is a coil of electrically resistive wire. The pitch of the coil is preferably between 0.5 and 1.5 mm, and most preferably approximately 1.5mm. The pitch of the coil means the spacing between adjacent turns of the coil. The coil may advantageously comprise fewer than six turns, and preferably has fewer than five turns. The electrically resistive wire advantageously has a diameter of between 0.10 and 0.15mm, and preferably of approximately 0.125mm. The electrically resistive wire is preferably formed of 904 or 301 stainless steel. Examples of other suitable metals include titanium, zirconium, tantalum and metals from the platinum group. Examples of other suitable metal alloys include, Constantan, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal®, iron-aluminium based alloys and iron-manganese-aluminium based alloys. Timetal® is a registered trade mark of Titanium Metals Corporation, 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required. The heating element may comprise a metallic etched foil insulated between two layers of an inert material. In that case, the inert material may comprise Kapton®, all-polyimide or mica foil. Kapton® is a registered trade mark of E.I. du Pont de Nemours and Company, 1007 Market Street, Wilmington, Delaware 19898, United States of America. The heating element may also comprise a metal foil, e.g., an aluminium foil, which is provided in the form of a ribbon.

The heating element may operate by resistive heating. In other words the material and dimensions of the heating element may be chosen so that when a particular current is passed through the heating element the temperature of the heating element is raised to a desired temperature. The current through the heating element may be applied by conduction from a battery or may be induced in the heating element by the application of a variable magnetic field around the heating element.

The heater and wick assembly may comprise more than one heating element, for example two, or three, or four, or five, or six or more heating elements.

The capillary body may comprise any suitable material or combination of materials which is able to convey a liquid aerosol-forming substrate along its length. The capillary body may be formed from a porous material, but this need not be the case. The capillary body may be formed from a material having a fibrous or spongy structure. The capillary body preferably comprises a bundle of capillaries. For example, the capillary body may comprise a plurality of fibres or threads or other fine bore tubes. The capillary body may comprise sponge-like or foam-like material. The structure of the capillary body forms a plurality of small bores or tubes, through which an aerosol-forming liquid can be transported by capillary action. The particular preferred material or materials will depend on the physical properties of the aerosol-forming substrate. Examples of suitable capillary materials include a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres, ceramic, glass fibres, silica glass fibres, carbon fibres, metallic fibres of medical grade stainless steel alloys such as austenitic 316 stainless steel and martensitic 440 and 420 stainless steels. The capillary body may have any suitable capillarity so as to be used with different liquid physical properties. The liquid has physical properties, including but not limited to viscosity, surface tension, density, thermal conductivity, boiling point and vapour pressure, which allow the liquid to be transported through the capillary body. The capillary body may be formed from heat-resistant material. Advantageously, the capillary body may comprise a plurality of fibre strands. The plurality of fibre strands may be generally aligned along the length of the capillary body.

Also described is a heater and wick assembly for an aerosol generating system, the assembly comprising: a capillary body; a heating element arranged on an outer surface of the capillary body; and a pair of spaced apart electrical contacts fixed around the capillary body and coupled with the heating element.

In a second aspect of the present invention, there is provided an aerosol generating system comprising: a heater and wick assembly according to any of the embodiments discussed above; a liquid storage portion in fluid communication with the capillary body; and an electric power supply connected to the heating element via the electrical contacts.

The electrical contacts may each comprise one or more outwardly extending tabs and the system may further comprise a housing having one or more ports into which the tabs are received and retained. This has the advantage that the tab of each electrical contact may allow the contact, and thus the heater and wick assembly, to be fastened easily to the housing and in the correct position. The one or more ports may comprise electrical connections connected to the electric power supply. With this arrangement, the tabs may facilitate electrical connection of the electrical contacts to the power supply. Preferably the one or more tabs are planar. The planar tabs provide a flat surface with which the heater and wick assembly may be located and retained within the aerosol-generating system. The flat surface of the planar tabs may also facilitate electrical connection of the electrical contacts to the power supply by presenting a larger electrically conductive surface area than with electrical contacts which do not have outwardly extending, planar tabs.

The aerosol generating system may be an electrically heated smoking system. Preferably, the aerosol-generating system is hand held. The aerosol-generating system may be an electrically heated smoking system and may have a size comparable to a conventional cigar or cigarette. The smoking system may have a total length between approximately 30 mm and approximately 150 mm. The smoking system may have an external diameter between approximately 5 mm and approximately 30 mm.

The system comprises a liquid storage portion in fluid communication with the capillary body of the heater and wick assembly. The liquid storage portion of the aerosol-generating system may comprise a housing that is substantially cylindrical, wherein an opening is at one end of the cylinder. The housing of the liquid storage portion may have a substantially circular cross section. The housing may be a rigid housing. As used herein, the term 'rigid housing' is used to mean a housing that is self-supporting. The liquid storage portion may contain an aerosol forming liquid.

The liquid storage portion may comprise a carrier material for holding the aerosol-forming substrate.

The liquid aerosol-forming substrate may be adsorbed or otherwise loaded onto a carrier or support. The carrier material may be made from any suitable absorbent plug or body, for example, a foamed metal or plastics material, polypropylene, terylene, nylon fibres or ceramic. The liquid aerosol-forming substrate may be retained in the carrier material prior to use of the aerosol-generating system. The liquid aerosol-forming substrate may be released into the carrier material during use. The liquid aerosol-forming substrate may be released into the carrier material immediately prior to use. For example, the liquid aerosol-forming substrate may be provided in a capsule. The shell of the capsule may melt upon heating by the heating means and releases the liquid aerosol-forming substrate into the carrier material. The capsule may optionally contain a solid in combination with the liquid.

In one example, the liquid aerosol-forming substrate is held in capillary material. A capillary material is a material that actively conveys liquid from one end of the material to another. The capillary material may be advantageously oriented in the storage portion to convey liquid aerosol-forming substrate to the heater and wick assembly. The capillary material may have a fibrous structure. The capillary material may have a spongy structure. The capillary material may comprise a bundle of capillaries. The capillary material may comprise a plurality of fibres. The capillary material may comprise a plurality of threads. The capillary material may comprise fine bore tubes. The capillary material may comprise a combination of fibres, threads and fine-bore tubes. The fibres, threads and fine-bore tubes may be generally aligned to convey liquid to the heater and wick assembly. The capillary material may comprise sponge-like material. The capillary material may comprise foam-like material. The structure of the capillary material may form a plurality of small bores or tubes, through which the liquid can be transported by capillary action.

The capillary material may comprise any suitable material or combination of materials. Examples of suitable materials are a sponge or foam material, ceramic- or graphite-based materials in the form of fibres or sintered powders, foamed metal or plastics materials, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different liquid physical properties. The liquid aerosol-forming substrate has physical properties, including but not limited to viscosity, surface tension, density, thermal conductivity, boiling point and atom pressure, which allow the liquid to be transported through the capillary material by capillary action. The capillary material may be configured to convey the aerosol-forming substrate to the atomiser.

The storage portion may comprise a fluid permeable internal surface surrounding an open-ended passage. The storage portion preferably comprises a capillary wick forming part or all of the internal surface for transporting aerosol-forming liquid from the storage portion to a heater assembly positioned within the open-ended passage.

The storage portion preferably contains an aerosol-forming liquid.

The liquid aerosol-forming substrate may comprise nicotine. The nicotine containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the aerosol-forming substrate upon heating. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise at least one aerosol-former. An aerosol-former is any suitable known compound or mixture of compounds that, in use, facilitates formation of a dense and stable aerosol and that is substantially resistant to thermal degradation at the temperature of operation of the system. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Aerosol formers may be polyhydric alcohols or mixtures thereof, such as triethylene glycol, 1,3-butanediol and glycerine. The liquid aerosol-forming substrate may comprise other additives and ingredients, such as flavourants.

The aerosol-forming substrate may comprise nicotine and at least one aerosol former. The aerosol former may be glycerine. The aerosol-former may be propylene glycol. The aerosol former may comprise both glycerine and propylene glycol. The aerosol-forming substrate may have a nicotine concentration of between about 2% and about 10%.

Although reference is made to liquid aerosol-forming substrates above, it will be clear to one of ordinary skill in the art that other forms of aerosol-forming substrate may be used with other embodiments. For example, the aerosol-forming substrate may be a solid aerosol-forming substrate. The aerosol-forming substrate may comprise both solid and liquid components. The aerosol-forming substrate may comprise a tobacco-containing material containing volatile tobacco flavour compounds which are released from the substrate upon heating. The aerosol-forming substrate may comprise a non-tobacco material. The aerosol-forming substrate may further comprise an aerosol former. Examples of suitable aerosol formers are glycerine and propylene glycol.

If the aerosol-forming substrate is a solid aerosol-forming substrate, the solid aerosol-forming substrate may comprise, for example, one or more of: powder, granules, pellets, shreds, spaghettis, strips or sheets containing one or more of: herb leaf, tobacco leaf, fragments of tobacco ribs, reconstituted tobacco, homogenised tobacco, extruded tobacco, cast leaf tobacco and expanded tobacco. The solid aerosol-forming substrate may be in loose form, or may be provided in a suitable container or cartridge. Optionally, the solid aerosol-forming substrate may contain additional tobacco or non-tobacco volatile flavour compounds, to be released upon heating of the substrate. The solid aerosol-forming substrate may also contain capsules that, for example, include the additional tobacco or non-tobacco volatile flavour compounds and such capsules may melt during heating of the solid aerosol-forming substrate.

As used herein, homogenised tobacco refers to material formed by agglomerating particulate tobacco. Homogenised tobacco may be in the form of a sheet. Homogenised tobacco material may have an aerosol-former content of greater than 5% on a dry weight basis. Homogenised tobacco material may alternatively have an aerosol former content of between 5% and 30% by weight on a dry weight basis. Sheets of homogenised tobacco material may be formed by agglomerating particulate tobacco obtained by grinding or otherwise comminuting one or both of tobacco leaf lamina and tobacco leaf stems. Alternatively, or in addition, sheets of homogenised tobacco material may comprise one or more of tobacco dust, tobacco fines and other particulate tobacco by-products formed during, for example, the treating, handling and shipping of tobacco. Sheets of homogenised tobacco material may comprise one or more intrinsic binders, that is tobacco endogenous binders, one or more extrinsic binders, that is tobacco exogenous binders, or a combination thereof to help agglomerate the particulate tobacco; alternatively, or in addition, sheets of homogenised tobacco material may comprise other additives including, but not limited to, tobacco and non-tobacco fibres, aerosol-formers, humectants, plasticisers, flavourants, fillers, aqueous and non-aqueous solvents and combinations thereof.

Optionally, the solid aerosol-forming substrate may be provided on or embedded in a thermally stable carrier. The carrier may take the form of powder, granules, pellets, shreds, spaghettis, strips or sheets. Alternatively, the carrier may be a tubular carrier having a thin layer of the solid substrate deposited on its inner surface, or on its outer surface, or on both its inner and outer surfaces. Such a tubular carrier may be formed of, for example, a paper, or paper like material, a non-woven carbon fibre mat, a low mass open mesh metallic screen, or a perforated metallic foil or any other thermally stable polymer matrix.

The solid aerosol-forming substrate may be deposited on the surface of the carrier in the form of, for example, a sheet, foam, gel or slurry. The solid aerosol-forming substrate may be deposited on the entire surface of the carrier, or alternatively, may be deposited in a pattern in order to provide a non-uniform flavour delivery during use.

The aerosol-generating system may consist of an aerosol generating device and a removable aerosol-generating article for use with the device. For example, the aerosol-generating article may comprise a cartridge or smoking article. The aerosol-generating article comprises the storage portion. The device may comprise a power supply and the electric circuitry. The heating and wick assembly may form part of the device or the article, or both the device and the article.

The system may comprise a cartridge removably coupled to an aerosol-generating device. The cartridge may be removed from the aerosol-generating device when the aerosol-forming substrate has been consumed. The cartridge may be disposable. The cartridge may be reusable. The cartridge may be refillable with liquid aerosol-forming substrate. The cartridge may be replaceable in the aerosol-generating device. The aerosol-generating device may be reusable. The cartridge may be manufactured at low cost, in a reliable and repeatable fashion. As used herein, the term removably coupled' is used to mean that the cartridge and device can be coupled and uncoupled from one another without significantly damaging either the device or cartridge. The cartridge may have a housing within which an aerosol-forming substrate is held. The cartridge may comprise a lid. The lid may be peelable before coupling the cartridge to the aerosol-generating device. The lid may be piercable.

Aerosol-generating systems according to the invention preferably comprise a housing and a heater assembly connected to the housing and comprising at least one heater and wick assembly according to any of the embodiments described above. The heater assembly preferably comprises an elongate support member connected to the housing and arranged to extend into the open-ended passage of a cartridge inserted in the cavity; and a plurality of heater and wick assemblies fixed to and spaced along the length of the elongate support member. The heater assembly may comprise a plurality of heater and wick assemblies. For example, the heater assembly may comprise two, three, four, five, six or more heater and wick assemblies fixed to and spaced along the length of the elongate support member.

The elongate support member may be formed by a hollow shaft portion defining an airflow passage forming part of an airflow pathway through the system. The at least one heater and wick assembly is preferably supported by the hollow shaft portion such that it extends across the airflow passage transverse to the longitudinal axis of the hollow shaft portion. In such embodiments, the at least one heater and wick assembly may span the airflow passage. Where one or more of the heater and wick assemblies extend across the airflow passage, the longitudinal axis of one or more of the heater and wick assemblies may be perpendicular to the longitudinal axis of the hollow shaft portion. One or more of the heater and wick assemblies extending across the airflow passage may be arranged such that its longitudinal axis is oblique to the longitudinal axis of the hollow shaft portion. Where the plurality of heater and wick assemblies extend across the airflow passage transverse to the longitudinal axis of the hollow shaft portion, one or more of the plurality of heater and wick assemblies may extend across the airflow passage such that its longitudinal axis is rotated about the longitudinal axis of the hollow shaft portion relative to the longitudinal axis of at least one other of the heater and wick assemblies. That is, when longitudinal axes of the heater and wick assemblies are projected onto a plane extending perpendicularly to the longitudinal axis of the hollow shaft portion, the longitudinal axis of one or more of the plurality of heater and wick assemblies may extend across the airflow passage at an angle to the longitudinal axis of at least one other of the heater and wick assemblies.

The elongate support member may be formed from an electrically conductive substrate, such as metal. The elongate support member may be formed from an electrically insulative substrate, such as a polymer substrate, and may further comprise one or more electrical conductors attached to the substrate for forming the heater and wick assemblies, for connecting the heater and wick assemblies to an electrical power source, or both. For example, the elongate support member may comprise an electrically insulative substrate on which electrical conductors are applied for example by deposition, printing, or by laminating with the substrate as a laminated foil. The laminate foil may then be shaped or folded to form the elongate support member.

The plurality of heater and wick assemblies may extend across the airflow passage transverse to the longitudinal axis of the hollow shaft portion. In such embodiments, the plurality of heater and wick assemblies may span the airflow passage.

Preferably, the hollow shaft portion comprises a plurality of apertures in which a plurality of heater and wick assemblies are held, the plurality of heater and wick assemblies being in fluid communication with the storage portion heater and wick assemblies through the plurality of apertures. The apertures may be formed in the hollow shaft portion after the hollow shaft portion has been formed, for example by punching, drilling, milling, erosion, electro erosion, cutting, or laser cutting. The apertures may be formed integrally with the hollow shaft portion at the time of forming the hollow shaft portion, for example by casting or moulding the hollow shaft portion with the apertures or by a deposition process, such as electrodeposition.

The elongate support member has a proximal end attached to the housing and a distal end downstream from the proximal end. In any of the embodiments described above, the elongate support member preferably has a piercing surface at its distal end. Thus, the elongate support member may double as an elongate piercing member. This may allow the elongate support member to conveniently and easily pierce a seal at the end of a cartridge during insertion of the cartridge into the device. To facilitate piercing of the seal, the distal end of the elongate support member at which the piercing surface is located preferably has a cross-sectional area that is smaller than the cross-sectional area of the region of the elongate support member immediately proximal of the piercing surface. Preferably, the cross-sectional area of the elongate support member narrows towards a tapered tip at the distal end of the elongate support member. The cross-sectional area of the elongate support member may narrow towards a point at the distal end of the elongate support member.

The aerosol-generating system may comprise an aerosol-forming chamber in which aerosol forms from a super saturated vapour, which aerosol is then carried into the mouth of a user. An air inlet, air outlet and the chamber are preferably arranged so as to define an airflow route from the air inlet to the air outlet via the aerosol-forming chamber, so as to convey the aerosol to the air outlet and into the mouth of a user.

The aerosol-generating system comprises an electrical power supply. The electrical power supply may be a battery. The battery may be a Lithium based battery, for example a Lithium-Cobalt, a Lithium-Iron-Phosphate, a Lithium Titanate or a Lithium-Polymer battery. The battery may be a Nickel-metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more smoking experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the heating means and actuator.

The aerosol-generating system may comprise a sensor for detecting an activation of the system. The sensor may comprise a puff detector in communication with electric circuitry in the system. The puff detector may be configured to detect when a user draws on the system. The electric circuitry may be configured to control power to the heating element in dependence on the input from the puff detector. The electric circuitry may comprise a microprocessor, which may be a programmable microprocessor, a microcontroller, or an application specific integrated chip (ASIC) or other electronic circuitry capable of providing control. The electric circuitry may comprise further electronic components. The electric circuitry may be configured to regulate a supply of power to the heater and wick assembly. Power may be supplied to the heater and wick assembly continuously following activation of the system or may be supplied intermittently, such as on a puff-by-puff basis. The power may be supplied to the heater and wick assembly in the form of pulses of electrical current.

The aerosol-generating system may comprise a user input, such as a switch or button. This may enable the user to turn the system on. The switch or button may activate the heater and wick assembly. The switch or button may initiate the aerosol generation. The switch or button may prepare electric circuitry to await input from a sensor, such as a puff sensor.

The aerosol-generating system may comprise a temperature sensor. The temperature sensor may be adjacent to the storage portion. The temperature sensor may be in communication with electric circuitry to enable the electric circuitry to maintain the temperature of the heating element at the predetermined operating temperature. The temperature sensor may be a thermocouple, or alternatively the heating element may be used to provide information relating to the temperature. The temperature dependent resistive properties of the heating element may be known and used to determine the temperature of the at least one heating element in a manner known to the skilled person.

The system may comprise a housing defining a cavity for receiving an aerosol-generating article, such as a consumable cartridge. The housing may comprise a main body and a mouthpiece portion. The cavity may be in the main body and the mouthpiece portion may have an outlet through which aerosol generated by the device can be drawn into a user's mouth. Alternatively, a mouthpiece portion may be provided as part of a cartridge for use with the device. As used herein, the term "mouthpiece portion" means a portion of the device or cartridge that is configured to be placed in a user's mouth in order to directly inhale an aerosol generated by the system, the aerosol being conveyed to the user's mouth through the mouthpiece. The heater and wick assembly may be connected to the main body or the mouthpiece portion.

Where the system comprises a housing defining a cavity for receiving an aerosol-generating article, the housing may be elongate. The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. Preferably, the material is light and non-brittle.

As used herein, the terms 'upstream' and 'downstream' are used to describe the relative positions of components, or portions of components, of aerosol-generating systems according to the invention in relation to the direction of air drawn through the aerosol-generating system during use thereof. Air is drawn into the system at its upstream end, passes downstream through the system and exits the system at its downstream end. The terms 'distal' and 'proximal', are used to describe the relative positions of components of aerosol-generating systems in relation to their connection to the rest of the system, such that the proximal end of a component is at the 'fixed' end which is connected to the system, and the distal end is at the 'free' end, opposite to the proximal end. Where a component is connected to the system at the downstream end of the component, the downstream end may be considered as the 'proximal' end, and vice versa. The upstream and downstream ends of the cartridge and the aerosol-generating device are defined with respect to the airflow when a user draws on the mouth end of the aerosol-generating device.

As used herein, the terms "longitudinal" and "length" refer to the direction between the opposed ends of a heater and wick assembly, or a component of an aerosol-generating system. The term "transverse" is used to describe the direction perpendicular to the longitudinal direction.

As used herein, the term "air inlet" is used to describe one or more apertures through which air may be drawn into the aerosol-generating system.

As used herein, the term "air outlet" is used to describe one or more aperture through which air may be drawn out of the aerosol-generating system.

In a third aspect of the present invention, there is provided a method of manufacturing a heater and wick assembly for an aerosol generating system, the method comprising the steps of: providing a capillary body, providing a support member extending along at least part of the length of the capillary body, arranging a heating element on an outer surface of the capillary body; and securing the heating element to the outer surface of the capillary body by fixing a pair of spaced apart electrical contacts around the capillary body and over the heating element.

The step of providing a capillary body may be carried out by providing a single length capillary body. The step of providing a support member extending along at least part of the length of the capillary body may be carried out by providing a single length support member. The step of arranging a heating element on an outer surface of the capillary body may be carried out by arranging a single length heating element on the single length capillary body. In such methods, each heater and wick assembly may be manufactured individually.

Preferably, the step of providing a capillary body is carried out by providing a multi-length capillary body, the step of arranging a heating element is carried out by arranging a multi-length heating element on an outer surface of the multi-length capillary body, and the step of securing the heating element is carried out by fixing a plurality of pairs of spaced apart electrical contacts around the multi-length capillary body and over the multi-length heating element to secure the multi-length heating element to the outer surface of the multi-length capillary body, and the method preferably further comprises the step of cutting the multi-length capillary body and the multi-length heating element between adjacent pairs of electrical contacts to form a plurality of heater and wick assemblies. In such embodiments, preferably the step of providing a support member is carried out by providing a multi-length support member, and the step of cutting also includes cutting the multi-length support member between adjacent pairs of electrical contacts.

Preferably, the step of securing the heating element is carried out by fixing one of the electrical contacts of each pair directly adjacent to one of the electrical contacts of the adjacent pair. Consequently, each pair is separated from the adjacent pair by only a small clearance. The step of cutting may then be carried out by cutting the multi-length capillary body and the multi-length heating element between the directly adjacent electrical contacts to form a plurality of heater and wick assemblies. The resulting heater and wick assemblies each have electrical contacts positioned at either end.

Also described is a method of manufacturing a heater and wick assembly for an aerosol generating system, the method comprising the steps of: providing a capillary body, arranging a heating element on an outer surface of the capillary body; and securing the heating element to the outer surface of the capillary body by fixing a pair of spaced apart electrical contacts around the capillary body and over the heating element.

Features described in relation to one or more aspects may equally be applied to other aspects of the invention. In particular, features described in relation to the heater and wick assembly of the first aspect may be equally applied to the aerosol-generating system of the second aspect, and vice versa, and features described in relation to either of the first and second aspects may equally apply to the method of manufacture of the third aspect.

The invention will be further described, by way of example only, with reference to the accompanying drawings in which:
Figure 1A shows a side view of a heater and wick assembly;
Figure 1B shows a transverse cross-sectional view of the heater and wick assembly of Figure 1A taken along line 1B-1B in Figure 1A;
Figures 1C to 1E show side views of first, second and third example electric contacts of the heater and wick assembly of Figure 1A, with the other components of the assembly removed for clarity;
Figure 1F shows a transverse cross-sectional view of an alternative heater and wick assembly;
Figure 2A shows a side view of a heater and wick assembly according to a first embodiment of the present invention;
Figure 2B shows a transverse cross-sectional view of the heater and wick assembly of Figure 2A taken along line 2B-2B in Figure 2A;
Figure 3A shows a side view of a heater and wick assembly according to a second embodiment of the present invention;
Figure 3B shows a transverse cross-sectional view of the heater and wick assembly of Figure 3A taken along line 3B-3B in Figure 3A;
Figure 4 shows a schematic longitudinal cross-section of an aerosol-generating system according to a first embodiment;
Figure 5 illustrates a longitudinal cross-section of a consumable cartridge for use with the aerosol-generating system of Figure 4;
Figure 6A shows a schematic longitudinal sectional view of the heater assembly of the aerosol-generating system of Figure 4;
Figure 6B illustrates a top view of the heater assembly of Figure 6A;
Figure 6C illustrates a side view of the heater assembly of Figure 6A; and
Figures 7A and 7B illustrate a method of inserting a consumable cartridge into the aerosol-generating device of the aerosol-generating system of Figure 4.

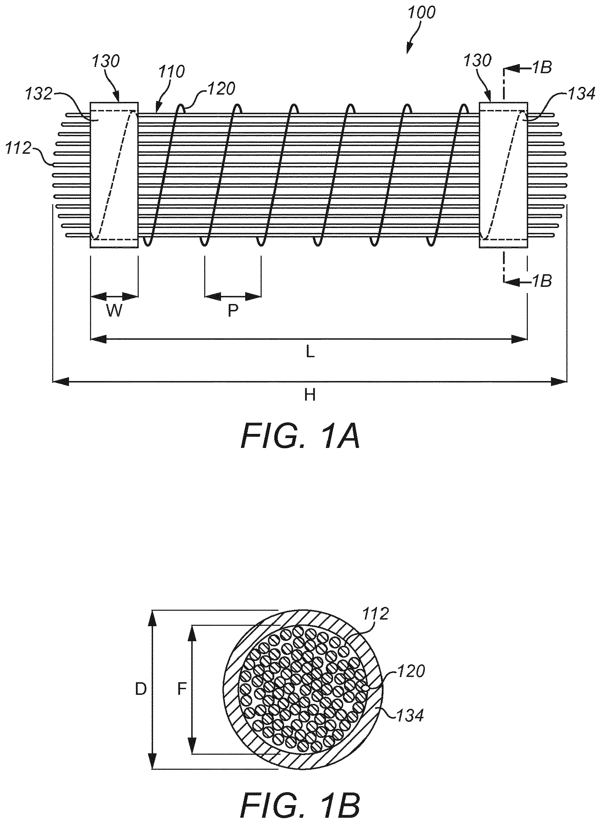

Figures 1A and 1B illustrate an example of a heater and wick assembly 100 for an aerosol-generating system. The heater and wick assembly 100 comprises a capillary body 110, a heating element 120 arranged on an outer surface of the capillary body 110, and a pair of spaced apart electrical contacts 130 fixed around the capillary body 110 and over the heating element 120.

The capillary body 110, or capillary wick, comprises a plurality of fibres 112 through which an aerosol-forming liquid can be transported by capillary action. In this example, the plurality of fibres 112 are generally aligned along the length of the capillary body 110. In other examples, the plurality of fibres may be woven or braided in a specific pattern. This allows the physical characteristics of the capillary wick, such as mechanical strength or capillarity, to be altered by using a particular pattern of fibres. It may also allow the capillary wick to maintain its shape and dimensions more effectively than with parallel fibres. The capillary body is compressible due to the existence of interstices between adjacent fibres. In this example, the capillary body 110 has rounded or domed end surfaces at both ends. This may help to increase the surface area between the capillary body 110 and an aerosol-forming liquid. In other examples, the capillary body 110 may terminate at flat end surfaces.

The heating element 120 is formed from a coil of electrically resistive wire wound around the capillary body 110 and extending along its entire length. The wire may have any suitable cross-sectional shape. In this example, the wire has a round cross-sectional shape. In other examples, the wire may have an oval, triangular, square, rectangular, or flat cross-sectional shape. This may increase heat transfer between the fibres 112 of the capillary body 110 and the wire of the heating element 120. The coil may have any suitable number of turns. For example, the coil may have from 2 to 11 full turns between the electrical contacts 130 at either end. Preferably, the coil has from 3 to 7 full turns between the electrical contacts 130.

The electrical contacts 130 comprise a first metallic ring 132 at a first end of the capillary body 110 and a second metallic ring 134 at a second end of the capillary body. The first and second metallic rings 132, 134 extend around the entire circumference of the capillary body 110 and over the heating element 120. The inner diameter of each of the metallic rings 132, 134 is less than the outer diameter of the capillary body 110. Consequently, there is an interference fit between the metallic rings 132, 134 and the capillary body 110 underneath. This ensures that the metallic rings 132, 134 press into the capillary body 110 to secure the rings 132, 134 to the capillary body, with the heating element 120 retained between. This helps to ensure a reliable electrical connection between the electrical contacts 130 and the heating element 120. As the electrical contacts 130 extend around the entire circumference of the capillary body 110, it is not necessary to carefully match the rotational position of the electrical contacts with the position of the heating coil 120 during assembly to ensure an electrical connection.

As shown in Figures 1A and 1B, the heater assembly 100 has the following dimensions. The dimension H is the total length, defined by the maximum length of the capillary body 110. The dimension L is the spacing between the first and second metallic rings, 132, 134. The dimension W is the width of the first and second metallic rings, 132, 134. The dimension D is the diameter of the heater assembly 100, defined by the diameter of the first and second metallic rings, 132, 134. The dimension F is the diameter of the capillary body 110. The dimension P is the pitch of the coil of the heating element 120.

Table 1 below illustrates example and preferred ranges for values of each of dimensions D, F, H, L, P and W, for such heater and wick assemblies.

**Table 1: Example and preferred dimension range values for heater and wick assemblies**

| Dimension | D | F | H | L | P | W |
|---|---|---|---|---|---|---|
| Example range (mm) | 1.4-4.5 | 1-4 | 4-12 | 3.5-11 | 0.5-2 | 0.7-2.5 |
| Preferred range (mm) | 1.6-2.8 | 1.3-2.5 | 5-8 | 4-7 | 0.6-1.1 | 0.8-1.3 |

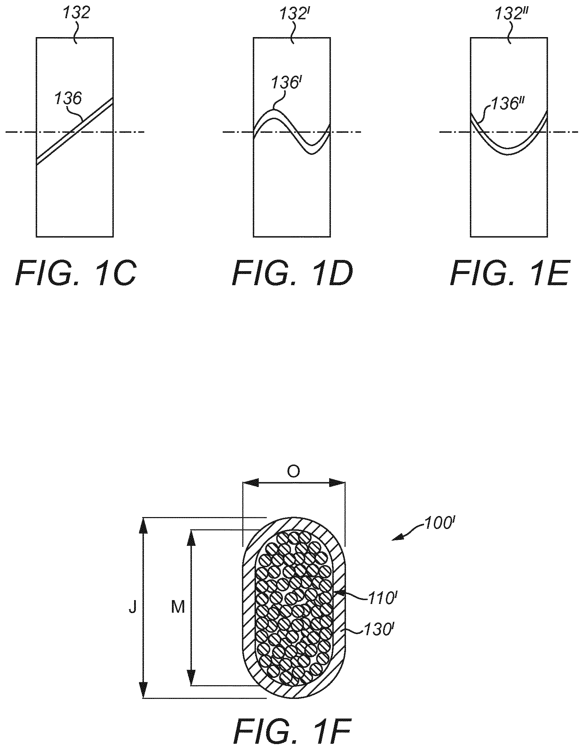

Figures 1C, 1D and 1E show a side view of three examples of metallic ring 132, 132', 132" for an electrical contact of the heater and wick assembly 100. In each of these examples, the electrical contacts 130 are rigid and formed from a bent sheet of metal, the opposed ends of which are connected together at a joint 136. The joint between the opposed ends of the metallic ring differs in each of the examples. As shown, the opposed ends of each electrical contact are co-operatively shaped such that the joint is non-linear or extends along an oblique line. This may help each of the electrical contacts to resist relative movement between its opposed ends in the length direction of the heater and wick assembly 100. In the example shown in Figure 1C, the opposed ends of the ring 132 are co-operatively shaped so that the joint 136 extends along a straight, oblique line. In the example shown in Figure 1D, the opposed ends of the ring 132' are co-operatively shaped so that the joint 136' is non-linear and has a wavy, or sinusoidal, shape. In the example shown in Figure 1E, the opposed ends of the ring 132" are co-operatively shaped so that the joint 136" is non-linear and has a parabolic, or U-, shape. It will be appreciated that other shapes of joint are envisaged, such as V-shaped, zig zag shaped, or curved.

In the examples shown in Figures 1A to 1E, the capillary body 110 has a circular cross-section and the electrical contacts 130 are in the form of circular rings. However, the capillary body 110 and electrical contacts may have any suitable cross-sectional shape. For example, the capillary body and electrical contacts may have an oval, triangular, square, rectangular, or lozenge-shaped cross-section, as shown in Figure 1F.

As shown in Figure 1F, the heater and wick assembly 100' has a lozenge-shaped cross-section as defined by a lozenge-shaped capillary body 110' and lozenge shaped electrical contacts 130'. As shown in Figures 1F, the heater assembly 100' has a height dimension J, a width dimension O, and a capillary body height dimension M which is equivalent to the height dimension J minus twice the thickness of the electric contact 130'. The dimensions J, M, and O may have any suitable value or range of values. For example, dimension J may have a value of from 1.4 to 5.5mm, preferably from 2.3 to 3.1mm, dimension M may have a value of from 1.3 to 5mm, preferably from 2 to 3mm, and dimension O may have a value of from 0.8 to 3mm, preferably from 0.8 to 2.2mm.

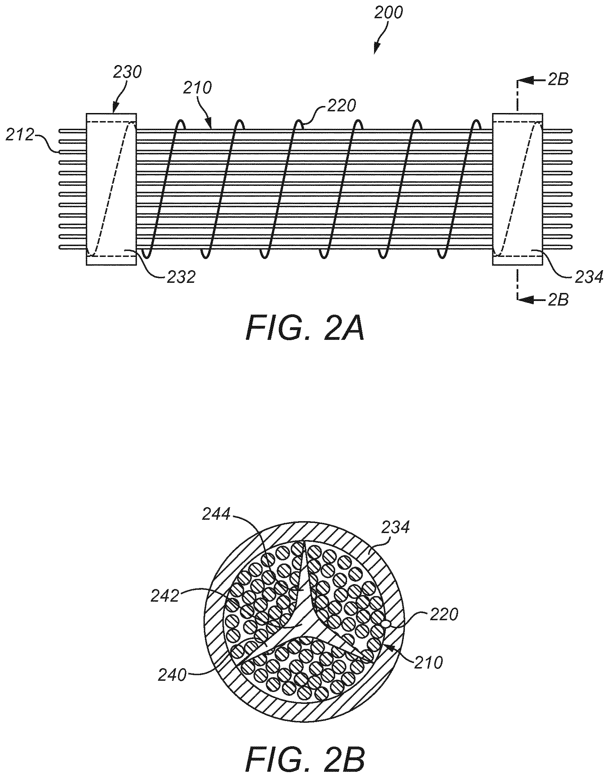

Figures 2A and 2B illustrate a first embodiment of a heater and wick assembly 200 for an aerosol-generating system. The heater and wick assembly 200 of the first embodiment has a similar structure to the example heater and wick assembly 100 and where the same features are present, like reference numerals have been used. However, the heater and wick assembly 200 further includes a rigid support member 240 extending through the core of the capillary body 210 and surrounded by the fibres 212 of the capillary body 210.

The support member 240 is a single, unitary component with a solid cross-section formed from a central portion 242 and a plurality of transverse ribs 244 extending radially from the central portion 242. This cross-sectional shape provides the support member 240 with a relatively high transverse rigidity for a given cross-sectional area. Due to this, the space within the capillary body which is occupied by the support member 240 may be minimised so that the wicking ability, or capillarity, of the capillary body 210 may be largely unaffected by the presence of the support member 240. The transvers ribs 244 are preferably tapered towards their tips. For example, each of the transverse ribs 244 may have a width at its base of from 0.3 to 0.8mm, preferably from 0.3 to 0.4mm, and a width at its base of from 0.1 to 0.4mm, preferably from 0.1 to 0.2mm.

The support member 240 extends along substantially the entire length of the capillary body 210 and is stronger and stiffer than the capillary body. Thus, the support member increases the strength and rigidity of the heater and wick assembly 200 to further improve robustness and ease of handling. In manufacturing operations in which individual heater and wick assemblies are cut from a multi-length heater and wick assembly, the support member may allow for improved accuracy of the cutting operation. This may lead to greater repeatability and consistency between different heater and wick assemblies.

In addition to increasing the bending strength and stiffness of the heater and wick assembly 200, the support member 240 also increases the density of the core of the capillary body 210. This may reduce the radial compressibility of the capillary body 210, thus helping to ensure a tight fit between the electrical contacts 230 and the heating element 220.

The support member 240 is formed from an electrical insulative material. This reduces the impact of the support member 240 on the electrical performance of the heating element 220 in the event of inadvertent contact between the heating element 220 and the support member 240.

The example and preferred dimensions of the heater assembly 200 of the first embodiment of the invention are the same as described above in relation to the first example heater assembly 100. As with the first example heater assembly, the coil of the heating element 220 may have any suitable number of turns, for example from 2 to 11 full turns between the electrical contacts 230, preferably from 3 to 7 full turns between the electrical contacts 230.

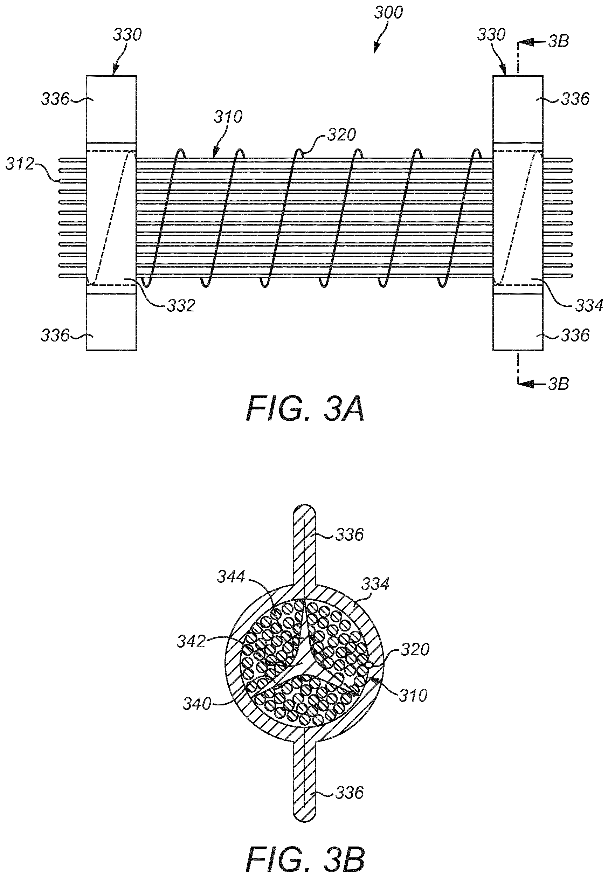

Figures 3A and 3B illustrate a second embodiment of a heater and wick assembly 300 for an aerosol-generating system. The heater and wick assembly 300 of the second embodiment has a similar structure to the heater and wick assembly 200 of the first embodiment and where the same features are present, like reference numerals have been used. However, unlike the example heater and wick assembly 100 and the first embodiment of heater and wick assembly 200, the electrical contacts 330 each have outwardly extending, planar tabs 336 on opposite sides of the heater and wick assembly 300. The tabs 336 provide a flat surface with which the heater and wick assembly 300 may be located and retained within an aerosol-generating system. For example, the tabs 336 may be received within one or more ports in an aerosol-generating system to allow the electrical contacts 330 to be fastened easily to the housing and in the correct position. The flat shape of the tabs 336 may also facilitate electrical connection of the electrical contacts to the power supply by presenting a larger electrically conductive surface area than with electrical contacts which do not have outwardly extending tabs.

The example and preferred dimensions of the heater assembly 300 are the same as described above in relation to the first example heater assembly 100 and the first embodiment of heater assembly 200. The coil of the heating element 320 may have any suitable number of turns, for example from 2 to 11 full turns between the electrical contacts 330, preferably from 3 to 7 full turns between the electrical contacts 330.

Heater and wick assemblies according to the present invention may be manufactured and assembled individually, for example by providing a single length capillary body and a single length support member, and arranging a single length heating element on the single length capillary body. Such a process may, for example, be carried out with the following steps. Step 1: Feed capillary fibres from a bobbin to form a continuous rod of fibres. Step 2A: Cut the continuous rod transversely to form a plurality of single length capillary bodies. Step 2B: Provide each of the plurality of single length capillary bodies with a single length support member extending along at least part of its length. Step 3: Unwind a length of electrically resistive wire from a bobbin and cut it to length. Step 4: Coil the cut length of wire around the single length capillary body and the support member to form the heating element. Step 5: Provide two sheets of electrically conductive material, either by unwinding from a bobbin and cutting to length or providing as a pre-cut segment. Step 6: Bend the sheets of electrically conductive material around the capillary body and over the heating element to form a spaced apart pair of electrical contacts in the form of clamping rings at either end of the capillary body. Step 7 (optional): Cut the capillary body to the correct length (if required) and shape one or both ends (if required, for example to provide rounded ends). Step 2B may be carried out before or after step 2A. For example, where step 2B is carried out before step 2A, step 2B may carried out by arranging a continuous support member extending along the length of the continuous rod. In such examples, step 2B may be carried out by cutting both the continuous rod and the continuous support member transversely to form a plurality of single length capillary bodies each having a support member extending along at least part of its length.

Heater and wick assemblies according to the present invention may be manufactured and assembled by providing multi-length capillary body, preferably having a length which is a multiple of the length of the capillary body of each heater and wick assembly, providing a multi-length support member, preferably having a length which is a multiple of the length of the support member of each heater and wick assembly, and providing a multi-length heating element, preferably having a length which is a multiple of the length of the heating element coil of each heater and wick assembly. This allows multiple heater and wick assemblies to be made more quickly. Such a process may, for example, be carried out with the following steps. Step 1A: Feed capillary fibres from a bobbin to form a continuous rod of fibres. Step 1B: Provide a continuous support member extending along the length of the continuous rod of fibres. Step 2: Feed a continuous electrically resistive wire from a bobbin and coil it around the continuous rod of fibres to form a continuous coil. Step 3: Provide a plurality of sheets of electrically conductive material, either by unwinding from a bobbin and cutting to length or providing as pre-cut segments. Step 4: Bend the sheets of electrically conductive material around the continuous rod of fibres and over the continuous coil to form a plurality of spaced apart pairs of electrical contacts in the form of clamping rings. Step 5: Cut the continuous rod of fibres, the continuous support member, and the continuous coil between adjacent pairs of electrical contacts to form a plurality of heater and wick assemblies. Step 6 (optional) shape one or both ends of each heater and wick assembly (if required, for example to provide rounded ends). Step 1B may be carried out before, after, or during step 1A.

Heater and wick assemblies according to the present invention may be manufactured in a fully automated process. The process may be carried out quickly and using standard equipment, such as that used in the pen industry and for electronics equipment. Using the processes described above, may allow assembly speeds of 4000 units / min.

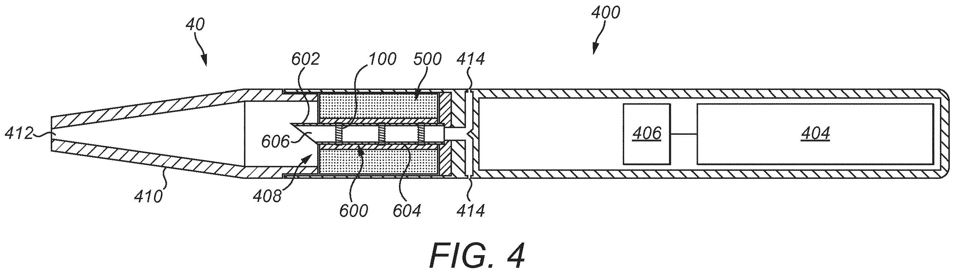

Figure 4 is a schematic illustration of an aerosol-generating system 40 incorporating a plurality of heater and wick assemblies according to the present invention comprising an aerosol-generating device 400 and an aerosol-generating article in the form of a consumable cartridge 500.

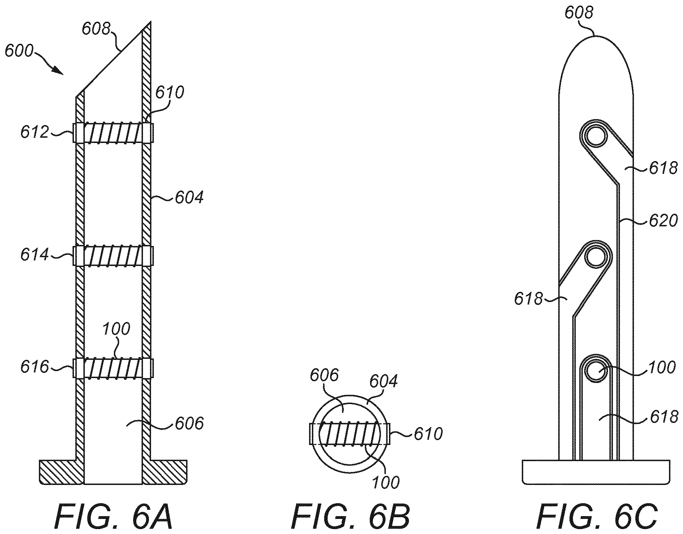

The device 400 comprises a main housing 402 containing a battery 404 and control electronics 406. The housing 402 also defines a cavity 408 into which the cartridge 500 is received. The device 400 further includes a mouthpiece portion 410 including an outlet 412. In this example, the mouthpiece portion 410 is connected to the main housing 402 by a screw fitting, but any suitable kind of connection may be used, such as a hinged connection or a snap fitting. The device 400 further includes a heater assembly 600 comprising an elongate support member in the form of an elongate piercing member 602 connected to the housing 402 and a plurality of heater and wick assemblies 100 according to the first embodiment of the invention. The plurality of heater and wick assemblies are each supported by the piercing member 602. The elongate piercing member 602 is positioned centrally within the cavity 408 of the device 400 and extends along the longitudinal axis of the cavity 408. The piercing member 602 comprises a hollow shaft portion 604 defining an airflow passage 606. Air inlets 414 are provided in the main housing 402 upstream of the heater assembly 600 and are in fluid communication with the outlet 412 via the airflow passage 606. The heater assembly is discussed in more detail below in relation to Figures 6A to 6C.

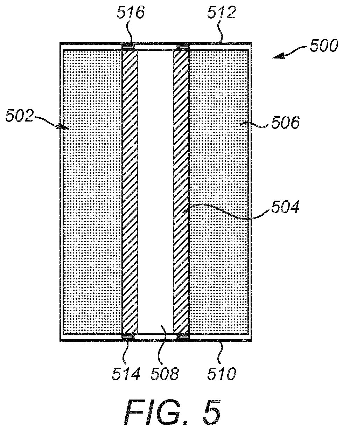

As best seen in Figure 5, the cartridge 500 comprises a storage portion 502 including a tubular capillary wick 504 surrounded by a tubular capillary material 506 containing liquid aerosol-forming substrate. The cartridge 500 has a hollow cylindrical shape through which extends an internal passageway 508. The capillary wick 504 surrounds the internal passageway 508 so that the internal passageway 508 is at least partly defined by an inner surface of the capillary wick 504. The upstream and downstream ends of the cartridge 500 are capped by frangible seals 510, 512. The cartridge 500 further includes a sealing ring 514, 516 at each of the upstream and downstream ends of the internal passageway 508.

As shown in Figures 6A, 6B and 6C, the hollow shaft portion 604 of the elongate piercing member 602 of the heater assembly 600 has a piercing surface 608 at its downstream end. In this example, the piercing surface 608 is formed by a sharp tip at the downstream end of the hollow shaft portion 604. The hollow shaft portion 604 has a plurality of apertures 610 within which the plurality of heater and wick assemblies 100 are held. The apertures 610 are provided in pairs, with each pair supporting a single electrical heater 100 at both of its ends. The two apertures in each pair are spaced apart around the circumference of the hollow shaft portion 604 so that each of the heater and wick assemblies 100 extends across the airflow passage 606. In this example, the plurality of apertures 610 comprises three pairs of apertures 612, 614, 616 supporting three heater and wick assemblies 100. The three pairs of apertures 612, 614, 616 are spaced apart along the length of the hollow shaft portion 604 and aligned around the circumference of the hollow shaft portion 604 such that the longitudinal axes of the three heater and wick assemblies 100 are parallel and rotationally aligned. It will be appreciated that other arrangements of heater assembly are envisaged. For example, the hollow shaft portion may include two or more pairs of apertures, for example three, four, five, six, or seven or more pairs of apertures. The pairs or apertures may be arranged such that the longitudinal axis of one or more of the heater and wick assemblies is rotated by any suitable amount, such as 90 degrees, about the longitudinal axis of the hollow shaft portion relative the longitudinal axis of one or more of the other heater and wick assemblies. In some examples, the heater and wick assemblies may be arranged in a helix or spiral around the hollow shaft portion.

The hollow shaft portion 604 is at least partially divided into a plurality of electrically isolated sections 618 which are electrically connected to the device 400. The apertures 610 in the hollow shaft portion 604 are each formed in one of the electrically isolated sections 618. In this manner, the heater and wick assemblies 100 held in the plurality of apertures 610 are electrically connected to the device 100. The electrically isolated sections 618 are electrically isolated from each other by insulating gaps 620. Thus, the heater and wick assemblies 100 may be electrically isolated from the each other to allow separate operation, control, or monitoring, without the need for separate electrical wiring for each heater. In this example, the gaps 620 are air gaps. That is, the gaps 620 do not contain insulating material. In other examples, one or more of the gaps 320 may be filled or partially filled with an electrically insulating material.

The electrical contacts of the heater and wick assemblies 100 and the apertures 610 in the piercing member 602 are co-operatively sized to provide a frictional fit. This ensures a secure fit between the hollow shaft portion 604 and the heater and wick assemblies 100. This may also enable a good electrical connection to be maintained between the heating element of each heater and wick assembly and the battery in the device 400. In this example, the apertures 610 are circular to match the shape of the electrical contacts of the heater and wick assemblies 100. In other examples, the cross-sectional shape of the electrical contacts may be different and the shape of the apertures determined accordingly. In examples where the heater and wick assemblies have outwardly extending tabs, as with the second embodiment of heater and wick assembly discussed above in relation to Figures 3A to 3C, the apertures 610 may have corresponding notches (not shown) which form ports into which the tabs may be received. Alternatively, or in addition, the piercing member 602 may include one or more clips in which the tabs may be located and retained.

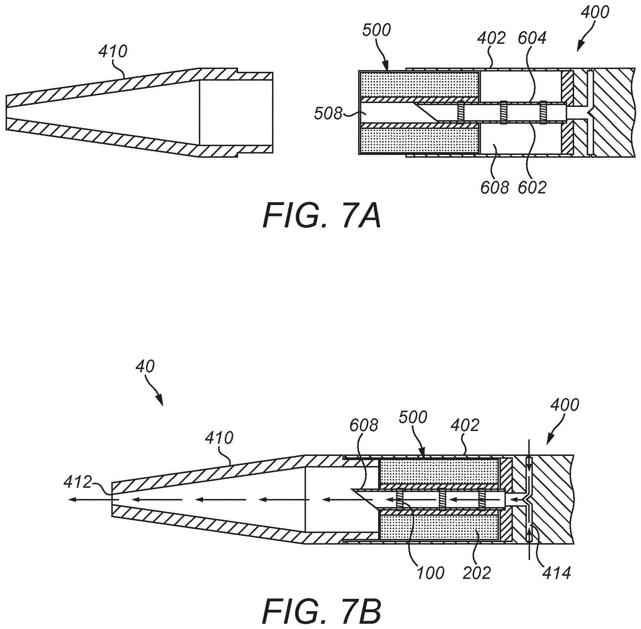

Referring to Figures 7A and 7B, insertion of the cartridge 500 into the device 400 of the system 40 is described. To insert the cartridge 500 into the device 400, and thereby assemble the system 40, the first step is to remove the mouthpiece portion 410 from the main housing 402 of the device 400 and to insert the article 500 into the cavity 408 of the device 400, as shown in Figure 7A. During insertion of cartridge 500 into the cavity 408, the piercing surface 608 at the distal end of the piercing member 602 breaks the frangible seal at the upstream end of the cartridge 500. As the cartridge 500 is inserted further into the cavity 408 and the piercing member 602 extends further into the internal passageway 508 of the cartridge, the piercing surface 608 engages with and breaks through the frangible seal at the downstream end of the cartridge 500 to create a hole in the frangible seal.

The cartridge 500 is then fully inserted into the cavity 408 and the mouthpiece portion 410 is replaced onto the main housing 402 and engaged thereto to enclose the cartridge 500 within the cavity 408, as shown in Figure 7B. When the cartridge 500 is fully inserted into the cavity 408, the holes in the frangible seals at the upstream and downstream ends of the cartridge 500 each have a diameter approximately equal to the outer diameter of the hollow shaft portion 604. The sealing rings at the upstream and downstream ends of the cartridge 500 form a seal around the hollow shaft portion 604. Together with the frangible seals this reduces or prevents leakage of liquid aerosol-forming substrate from the cartridge 500 and out of the system 40. The cartridge 500 may be pressed fully into the cavity 408 by the user before the mouthpiece portion 410 is replaced onto the main housing 402. Alternatively, the cartridge 500 may be partially inserted into the cavity 408 and the mouthpiece portion 410 used to push the cartridge 500 into the cavity 408 until it is fully inserted. This may be more convenient for the user.

As shown in Figure 7B, when the cartridge 500 is fully inserted into the cavity 408 of the aerosol-generating device 400, an airflow pathway, shown by arrows in Figure 7B, is formed through the aerosol-generating system 40. The airflow pathway extends from the air inlets 414 to the outlet 412 via the internal passageway 508 in the cartridge 500 and the airflow passage 606 in the heater assembly 600. As also shown in Figure 7B, when the cartridge 500 is fully inserted, the heater and wick assemblies 100 are in fluid communication with the storage portion 502 of the cartridge 500 at the inner surface of the internal passageway 508.

In use, liquid aerosol-forming substrate is transferred from the storage portion 502 to the capillary body of each heater and wick assembly 100 via capillary action and through the plurality of apertures in the piercing member 602. In this example, the outer diameter of the hollow shaft portion 604 of the elongate piercing member 602 is greater than the inner diameter of the internal passageway 508 of the cartridge 500 so that the storage portion 502 of the cartridge 500 is compressed by the hollow shaft portion 604. This ensures direct contact between the ends of the heater and wick assemblies 100 and the storage portion 502 to help transfer of liquid aerosol-forming substrate to the heater and wick assemblies 100. The battery supplies electrical energy to the heating element of each heater and wick assembly 100, via the piercing member 602 and the electrical contacts of each heater and wick assembly 100. The heating elements heat up to vaporise liquid substrate in the capillary body of the heater and wick assemblies 100 to create a supersaturated vapour. At the same time, the liquid being vaporised is replaced by further liquid moving along the capillary wick of the liquid storage portion 502 and the capillary body of each heater and wick assembly 100 by capillary action. (This is sometimes referred to as "pumping action".) When a user draws on the mouthpiece portion 410, air is drawn through the air inlets 414, through the airflow passage of the hollow shaft portion 604, past the heater and wick assemblies 100, into the mouthpiece portion 410 and out of the outlet 412. The vaporised aerosol-forming substrate is entrained in the air flowing through the airflow passage of the hollow shaft portion 604 and condenses within the mouthpiece portion 410 to form an inhalable aerosol, which is carried towards the outlet 412 and into the mouth of a user.

The device may be operated by a user-operated switch (not shown) on the device 400. Alternatively, or in addition, the device may include a sensor for detecting a user puff. When a puff is detected by the sensor, the control electrics control the supply of electrical energy from the battery to the heater and wick assemblies 100. The sensor may comprise one or more separate components. In some examples, the puff sensing function is performed by the heating elements of the heater and wick assemblies. For example, by measuring with the control electronics one or more electrical parameters of the heating elements and detecting a particular change in the measured electrical parameters which is indicative of a puff.

## Claims

1. A heater and wick assembly (200) for an aerosol generating system (40), the assembly comprising:
a capillary body (210);
a heating element (220) arranged on an outer surface of the capillary body (210);
a pair of spaced apart electrical contacts (230) fixed around the capillary body (210) and coupled with the heating element (220); and
a support member (240) extending along at least part of the length of the capillary body (210).

2. A heater and wick assembly (200) according to claim 1, wherein at least one of the electrical contacts (230) is dimensioned such that there is a frictional fit between an inner surface of that electrical contact and the outer surface of the capillary body (210).

3. A heater and wick assembly (200) according to any preceding claim, wherein at least one of the electrical contacts (230) extends around at least part of the circumference of the capillary body (210) and is dimensioned such that there is an interference fit between the electrical contact and the capillary body (210).

4. A heater and wick assembly (200) according to any preceding claim, wherein one or both of the electrical contacts (230) extend around substantially the entire circumference of the capillary body (210).

5. A heater and wick assembly (200) according to any preceding claim, wherein one or both of the electrical contacts (230) is rigid.

6. A heater and wick assembly (200) according to any preceding claim, wherein the heating element (220) comprises a coil of electrically resistive wire wound around the capillary body (210).

7. A heater and wick assembly (200) according to any preceding claim, wherein the capillary body (210) is elongate and the pair of electrical contacts (230) comprises a first electrical contact at or adjacent to a first end of the capillary body (210) and a second electrical contact at or adjacent to the second end of the capillary body (210).

8. A heater and wick assembly (200) according to any preceding claim, wherein the support member (240) is rigid.

9. A heater and wick assembly (200) according to any preceding claim, wherein the support member (240) extends along substantially the entire length of the capillary body (210).

10. A heater and wick assembly (200) according to any preceding claim, wherein the support member (240) has a solid cross-sectional area.

11. A heater and wick assembly (200) according to any preceding claim, wherein the support member (240) comprises a central portion and a plurality of transverse ribs.

12. A heater and wick assembly (200) according to any preceding claim, wherein the support member (240) is formed from an electrical insulative material.

13. An aerosol-generating system (40) comprising:
a heater and wick assembly (200) according to any of the preceding claims;
a liquid storage portion (502) in fluid communication with the capillary body (210); and
an electric power supply connected to the heating element (220) via the electrical contacts (230).

14. An aerosol-generating system (40) according to claim 13, wherein the electrical contacts (230) each comprise an outwardly extending tab (336) and the system further comprises a housing (402) having one or more ports into which one or both of the outwardly extending tabs (336) are received and retained.

15. An aerosol-generating system (40) according to claim 13 or claim 14, wherein the aerosol-generating system (40) is an electrically heated smoking system.

16. A method of manufacturing a heater and wick assembly (200) for an aerosol generating system (40), the method comprising the steps of:
providing a capillary body (210);
providing support member (240) extending along at least part of the length of the capillary body (210);
arranging a heating element (220) on an outer surface of the capillary body (210); and
securing the heating element (220) to the outer surface of the capillary body (210) by fixing a pair of spaced apart electrical contacts (230) around the capillary body (210) and over the heating element (220).

17. A method of manufacturing a heater and wick assembly (200) for an aerosol generating system (40) according to claim 16, wherein:
the step of providing a capillary body (210) is carried out by providing a multi-length capillary body,
the step of arranging a heating element (220) is carried out by arranging a multi-length heating element on an outer surface of the multi-length capillary body, and
the step of securing the heating element (220) is carried out by fixing a plurality of pairs of spaced apart electrical contacts (230) around the multi-length capillary body and over the multi-length heating element to secure the multi-length heating element to the outer surface of the multi-length capillary body, and
wherein the method further comprises the step of cutting the multi-length capillary body and the multi-length heating element between adjacent pairs of electrical contacts (230) to form a plurality of heater and wick assemblies (100).

## Patentansprüche

1. Heizvorrichtungs- und Dochtanordnung (200) für ein Aerosolerzeugungssystem (40), wobei die Anordnung aufweist:
einen Kapillarkörper (210);
ein Heizelement (220), das auf einer Außenfläche des Kapillarkörpers (210) angeordnet ist;
ein Paar beabstandeter elektrischer Kontakte (230), die um den Kapillarkörper (210) herum befestigt und mit dem Heizelement (220) gekoppelt sind; und
ein Stützelement (240), das sich mindestens über einen Teil der Länge des Kapillarkörpers (210) erstreckt.

2. Heizvorrichtungs- und Dochtanordnung (200) nach Anspruch 1, wobei mindestens einer der elektrischen Kontakte (230) derart dimensioniert ist, dass zwischen einer Innenfläche dieses elektrischen Kontakts und der Außenfläche des Kapillarkörpers (210) ein Kraftschluss besteht.

3. Heizvorrichtungs- und Dochtanordnung (200) nach einem der vorstehenden Ansprüche, wobei sich mindestens einer der elektrischen Kontakte (230) um mindestens einen Teil des Umfangs des Kapillarkörpers (210) herum erstreckt und derart dimensioniert ist, dass zwischen dem elektrischen Kontakt und dem Kapillarkörper (210) eine Presspassung besteht.

4. Heiz- und Dochtanordnung (200) nach einem der vorstehenden Ansprüche, wobei sich einer oder beide der elektrischen Kontakte (230) im Wesentlichen um den gesamten Umfang des Kapillarkörpers (210) herum erstrecken.

5. Heizvorrichtungs- und Dochtanordnung (200) nach einem der vorstehenden Ansprüche, wobei einer oder beide der elektrischen Kontakte (230) starr sind.

6. Heizvorrichtungs- und Dochtanordnung (200) nach einem der vorstehenden Ansprüche, wobei das Heizelement (220) eine Spule aus elektrischem Widerstandsdraht aufweist, die um den Kapillarkörper (210) herum gewickelt ist.

7. Heizvorrichtungs- und Dochtanordnung (200) nach einem der vorstehenden Ansprüche, wobei der Kapillarkörper (210) länglich ist und das Paar von elektrischen Kontakten (230) einen ersten elektrischen Kontakt an oder nahe einem ersten Ende des Kapillarkörpers (210) und einen zweiten elektrischen Kontakt an oder nahe dem zweiten Ende des Kapillarkörpers (210) aufweist.

8. Heizvorrichtungs- und Dochtanordnung (200) nach einem der vorstehenden Ansprüche, wobei das Stützelement (240) starr ist.

9. Heizvorrichtungs- und Dochtanordnung (200) nach einem der vorstehenden Ansprüche, wobei sich das Stützelement (240) im Wesentlichen entlang der gesamten Länge des Kapillarkörpers (210) erstreckt.

10. Heizvorrichtungs- und Dochtanordnung (200) nach einem der vorstehenden Ansprüche, wobei das Stützelement (240) eine feste Querschnittsfläche aufweist.

11. Heizvorrichtungs- und Dochtanordnung (200) nach einem der vorstehenden Ansprüche, wobei das Stützelement (240) einen zentralen Abschnitt und mehrere Querrippen aufweist.

12. Heizvorrichtungs- und Dochtanordnung (200) nach einem der vorstehenden Ansprüche, wobei das Stützelement (240) aus einem elektrisch isolierenden Material gebildet ist.

13. Aerosolerzeugungssystem (40), aufweisend:
eine Heizvorrichtungs- und Dochtanordnung (200) nach einem der vorstehenden Ansprüche;
einen Flüssigspeicherteil (502) in Fluidverbindung mit dem Kapillarkörper (210); und
eine elektrische Stromversorgung, die über die elektrischen Kontakte (230) mit dem Heizelement (220) verbunden ist.

14. Aerosolerzeugungssystem (40) nach Anspruch 13, wobei die elektrischen Kontakte (230) jeweils eine sich nach außen erstreckende Lasche (336) aufweisen und das System weiter ein Gehäuse (402) mit einer oder mehreren Öffnungen aufweist, in denen eine oder beide der sich nach außen erstreckenden Laschen (336) aufgenommen sind und zurückgehalten werden.

15. Aerosolerzeugungssystem (40) nach Anspruch 13 oder Anspruch 14, wobei das Aerosolerzeugungssystem (40) ein elektrisch beheiztes System zum Rauchen ist.

16. Verfahren zur Fertigung einer Heizvorrichtungs- und Dochtanordnung (200) für ein Aerosolerzeugungssystem (40), wobei das Verfahren die Schritte aufweist:
Bereitstellen eines Kapillarkörpers (210);
Bereitstellen des Stützelements (240), das sich mindestens über einen Teil der Länge des Kapillarkörpers (210) erstreckt;
Anordnen eines Heizelements (220) auf einer Außenfläche des Kapillarkörpers (210); und
Befestigen des Heizelements (220) an der Außenfläche des Kapillarkörpers (210) durch Befestigen eines Paars von beabstandeten elektrischen Kontakten (230) um den Kapillarkörper (210) herum und über das Heizelement (220).

17. Verfahren zum Herstellen einer Heizvorrichtungs- und Dochtanordnung (200) für ein Aerosolerzeugungssystem (40) nach Anspruch 16, wobei:
der Schritt des Bereitstellens eines Kapillarkörpers (210) durch Bereitstellen eines Mehrlängenkapillarkörpers erfolgt,
der Schritt das Anordnen eines Heizelements (220) durch Anordnen eines Mehrlängenheizelements auf einer Außenfläche des Mehrlängenkapillarkörpers erfolgt und
der Schritt des Befestigens des Heizelements (220) durch Befestigen von mehreren Paaren von beabstandeten elektrischen Kontakten (230) um den Mehrlängenkapillarkörper herum und über das Mehrlängenheizelement erfolgt, um das Mehrlängenheizelement an der Außenfläche des Mehrlängenkapillarkörpers zu befestigen, und
wobei das Verfahren weiter den Schritt des Trennens des Mehrlängenkapillarkörpers und des Mehrlängenheizelements zwischen benachbarten Paaren von elektrischen Kontakten (230), um mehrere Heizvorrichtungs- und Dochtanordnungen (100) zu bilden, aufweist.

## Revendications

1. Ensemble de chauffage et de mèche (200) pour un système de génération d'aérosol (40), l'ensemble comprenant :
un corps capillaire (210) ;
un élément de chauffage (220) agencé sur une surface externe du corps capillaire (210) ;
une paire de contacts électriques espacés (230) fixés autour du corps capillaire (210) et couplés à l'élément de chauffage (220) ; et
un élément de support (240) s'étendant le long au moins d'une partie de la longueur du corps capillaire (210).

2. Ensemble de chauffage et de mèche (200) selon la revendication 1, dans lequel au moins un des contacts électriques (230) est dimensionné de telle sorte qu'il existe un ajustement de friction entre une surface interne de ce contact électrique et la surface externe du corps capillaire (210).

3. Ensemble de chauffage et de mèche (200) selon l'une quelconque des revendications précédentes, dans lequel au moins un des contacts électriques (230) s'étend autour d'au moins une partie de la circonférence du corps capillaire (210) et est dimensionné de telle sorte qu'il existe un ajustement d'interférence entre le contact électrique et le corps capillaire (210).

4. Ensemble de chauffage et de mèche (200) selon l'une quelconque des revendications précédentes, dans lequel un ou les deux contacts électriques (230) s'étendent autour de sensiblement la circonférence entière du corps capillaire (210).

5. Ensemble de chauffage et de mèche (200) selon l'une quelconque des revendications précédentes, dans lequel un ou les deux contacts électriques (230) sont rigides.

6. Ensemble de chauffage et de mèche (200) selon l'une quelconque des revendications précédentes, dans lequel l'élément de chauffage (220) comprend une bobine de bobinage électrique résistif autour du corps capillaire (210).

7. Ensemble de chauffage et de mèche (200) selon l'une quelconque des revendications précédentes, dans lequel le corps capillaire (210) est allongé et la paire de contacts électriques (230) comprend un premier contact électrique sur ou contre une première extrémité du corps capillaire (210) et un deuxième contact électrique sur ou contre la deuxième extrémité du corps capillaire (210).

8. Ensemble de chauffage et de mèche (200) selon l'une quelconque des revendications précédentes, dans lequel l'élément de support (240) est rigide.

9. Ensemble de chauffage et de mèche (200) selon l'une quelconque des revendications précédentes, dans lequel l'élément de support (240) s'étend le long de sensiblement toute la longueur du corps capillaire (210).

10. Ensemble de chauffage et de mèche (200) selon l'une quelconque des revendications précédentes, dans lequel l'élément de support (240) possède une zone de coupe transversale solide.

11. Ensemble de chauffage et de mèche (200) selon l'une quelconque des revendications précédentes, dans lequel l'élément de support (240) comprend une partie centrale et une pluralité de nervures transversales.

12. Ensemble de chauffage et de mèche (200) selon l'une quelconque des revendications précédentes, dans lequel l'élément de support (240) est formé d'un matériau isolant électrique.

13. Système de génération d'aérosol (40) comprenant :
un ensemble de chauffage et de mèche (200) selon l'une quelconque des revendications précédentes ;
une partie de stockage liquide (502) en communication fluide avec le corps capillaire (210) ; et
une alimentation électrique connectée à l'élément de chauffage (220) via les contacts électriques (230) .

14. Système de génération d'aérosol (40) selon la revendication 13, dans lequel les contacts électriques (230) comprennent chacun une languette s'étendant vers l'extérieur (336) et le système comprend en outre un logement (402) ayant un ou plusieurs ports dans lesquels un ou les deux des languettes s'étendant vers l'extérieur (336) sont reçues et conservées.

15. Système de génération d'aérosol (40) selon la revendication 13 ou la revendication 14, dans lequel le système de génération d'aérosol (40) est un système à fumer chauffé électriquement.

16. Procédé de fabrication d'un ensemble de chauffage et de mèche (200) pour un système de génération d'aérosol (40), le procédé comprenant les étapes de :
la fourniture d'un corps capillaire (210) ;
la fourniture d'un élément de support (240) s'étendant le long d'au moins une partie de la longueur du corps capillaire (210) ;
l'agencement d'un élément de chauffage (220) sur une surface externe du corps capillaire (210) ; et
la fixation de l'élément de chauffage (220) à la surface externe du corps capillaire (210) en fixant une paire de contacts électriques espacés (230) autour du corps capillaire (210) et sur l'élément de chauffage (220).

17. Procédé de fabrication d'un ensemble de chauffage et de mèche (200) pour un système de génération d'aérosol (40) selon la revendication 16, dans lequel :
l'étape consistant de fourniture d'un corps capillaire (210) est réalisée par la fourniture d'un corps capillaire multi-longueur,
l'étape de l'agencement d'un élément de chauffage (220) est réalisée par l'agencement d'un élément de chauffage multi-longueurs sur une surface externe du corps capillaire multi-longueur, et
l'étape de fixation de l'élément de chauffage (220) est réalisée en fixant une pluralité de paires de contacts électriques espacés (230) autour du corps capillaire multi-longueur et sur l'élément de chauffage multi-longueurs pour fixer l'élément de chauffage multi-longueurs à la surface externe du corps capillaire multi-longueur, et
dans lequel le procédé comprend en outre l'étape de coupe du corps capillaire multi-longueur et l'élément de chauffage multi-longueurs entre les paires adjacentes de contacts électriques (230) pour former une pluralité d'ensembles de chauffage et de mèche (100).
